# EUROPEAN PATENT APPLICATION

(11) **EP 1 669 347 A1**
(43) Date of publication of application: **14.06.2006**
(21) Application number: 04029364.9
(22) Date of filing: 10.12.2004
(51) Int. Cl.: C07C 315/00, C07C 315/04, C07C 317/46, C07C 59/115

(54) **Process for the preparation of 3-¬(4-fluorophenyl) sulfonyl|-2-hydroxy-2-methyl propionic acid**

(71) Applicant: Helm AG, 20097 Hamburg (DE); CF Pharma Gyogyszergyarto Kft., 1097 Budapest (HU)
(72) Inventor: Bor, Adam, 2040 Budaörs (HU); Orosz, György, 1143 Budapest (HU); Lukacs, Ferenc, 2143 Kistarcsa (HU); Schneider, Géza, 1025 Budapest (HU)
(74) Representative: Teipel, Stephan

(57) **Abstract**

The present invention relates to a process for the preparation of 3-[(4-fluorophenyl)sulfonyl]-2-hydroxy-2-methylpropionic acid, which comprises the step of reacting 3-chloro-2-methyl-2-hydroxypropionic acid with a salt of 4-fluorophenylsulfinic acid, and the use of (+/-)- and (-)-3-[(4-fluorophenyl)sulfonyl]-2-hydroxy-2-methylpropionic acid and (+)-3-chloro-2-methyl-2-hydroxypropionic acid in the preparation of bicalutamide.

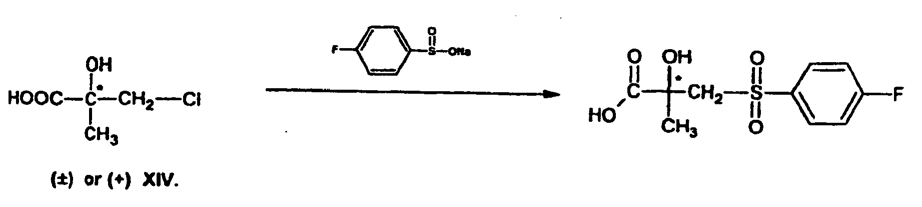

## Description

The present invention relates to a process for the preparation of 3-[(4-fluorophenyl)sulfonyl]-2-hydroxy-2-methyl propionic acid and to the production of bicalutamide (formula I in Scheme 1).

Bicalutamide ((R,S)-N-[4-cyano-3-(trifluoromethyl)phenyl]-3-(4-fluorophenylsulfonyl)-2-hydroxy-2-methyl-propanamide) is a highly efficient antiandrogen containing a β-hydroxy-sulfone moiety. It is effective in treating malign and benign prostate cancer.

Bicalutamide contains one chiral carbon atom. According to the clinical trials, the beneficial pharmacological activity can be attributed mainly to the R(-)-enantiomer (Tucker, H. et al.: J.Med.Chem. 31 (1988) 885-7).

There are several processes known in the art for producing bicalutamide, which are summarized in Scheme 1.

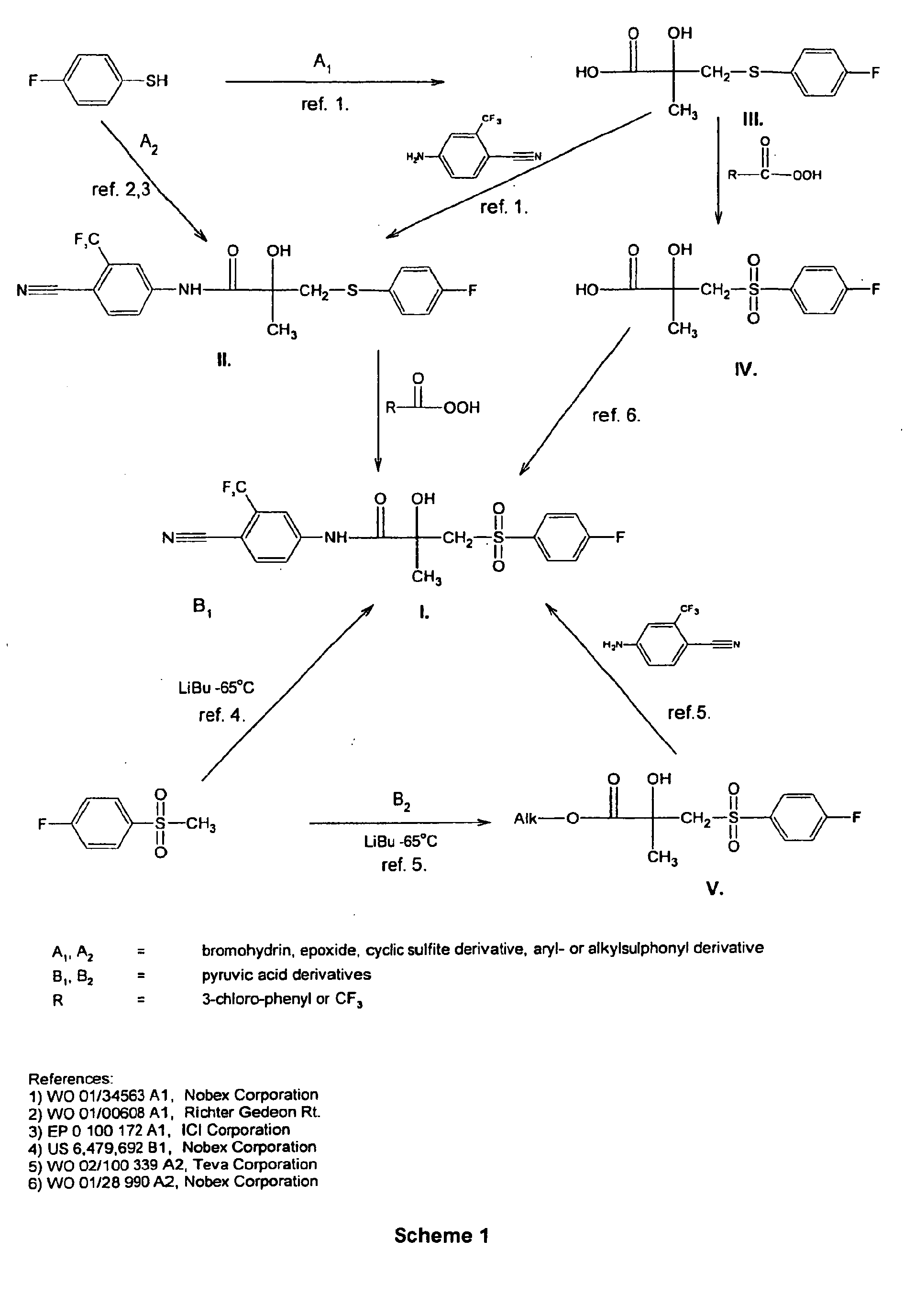

In the first type of bicalutamide production thioethers **II** and **III** obtained from 4-fluoro-thiophenol are oxidized by a peracid to yield bicalutamide I. The disadvantages of such processes are that 1) the toxic and very stinky 4-fluoro-thiophenol is used in the production and 2) oxidation of the thioethers obtained (**II** or **III**) by peracids (e.g. m-chloro-perbenzoic acid or trifluoroperacetic acid) are expensive and explosive in plant scale.

In another type of bicalutamide synthesis pyruvic acid derivatives (B₁, B₂) are reacted with a lithiated sulfon. The disadvantage of such processes is that 1) pyruvic acid and its derivatives are unstable, 2) the lithium compounds are expensive and require special handling and 3) working in plant scale at around -65°C due to the requirements of the lithiation is a difficult technological problem. A further disadvantage is that in such type of process only racemic bicalutamide can be produced.

The known processes can be classified into three types of processes, two reaction variations of which are summarized in Scheme 2. Only the sequence of the reaction steps is different in various reaction sequences, e.g., synthesis of **I** from **IV**.

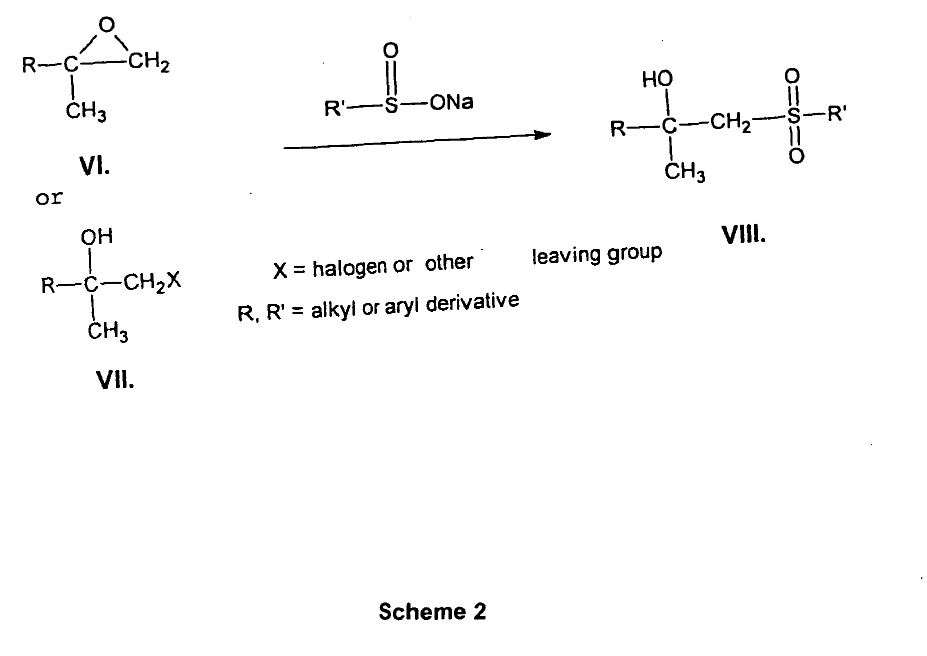

It is known from the art (e.g., Houben-Weyl Band E 11, Teil 2, 1145-1164) that β-hydroxy-sulfone derivatives described by the general formula **VIII** can be synthesized via a nucleophilic displacement reaction in the reaction of sulfinate salts and a) epoxides of the general formula **VI,** or b) halohydrines or compounds containing other leaving groups instead of halogen of the general formula **VII** (cf. Scheme 2).

Such reactions are described in PCT/EP03/04999 and US 2003/0073742 according to which suitably substituted derivatives can be prepared via chlorohydrin or epoxy derivatives (Scheme 3).

As it is shown in Scheme 3, the final step of bicalutamide synthesis can be either the reaction of acyl anilide **(IX)** or epoxide **(X)** and 4-fluorophenyl sulfinate, or the reaction of 3-[(4-fluorophenyl)sulfonyl]-2-hydroxy-2-methyl propionic acid **(XIII, R=H)** with 3-amino-2-trifluoromethyl benzonitrile. From the point of view of product cost and efficiency, the second reaction route is more advantageous.

According to US 2003/0073742 the reaction of sulfinate with protected halohydrine and epoxy esters proceed with modest yield (31%). In the reaction route described in US 2003/0073742 the starting epoxy or halohydrine esters are expensive compounds and the derivatives obtained should be hydrolyzed to obtain the acid for the economy of the process. If compound **XIII** is an acid then thionyl chloride activation can be used in the synthesis of bicalutamide (Tucker et al., ref. **12).**

One problem of the present invention was to provide a cost-efficient synthesis of 3-[(4-fluorophenyl)sulfonyl]-2-hydroxy-2-methyl propionic acid (**XIII, R=H**) which can be reacted directly with 4-amino-2-trifluoromethyl benzonitrile following the general procedure described by Tucker et al. (ref. 12).

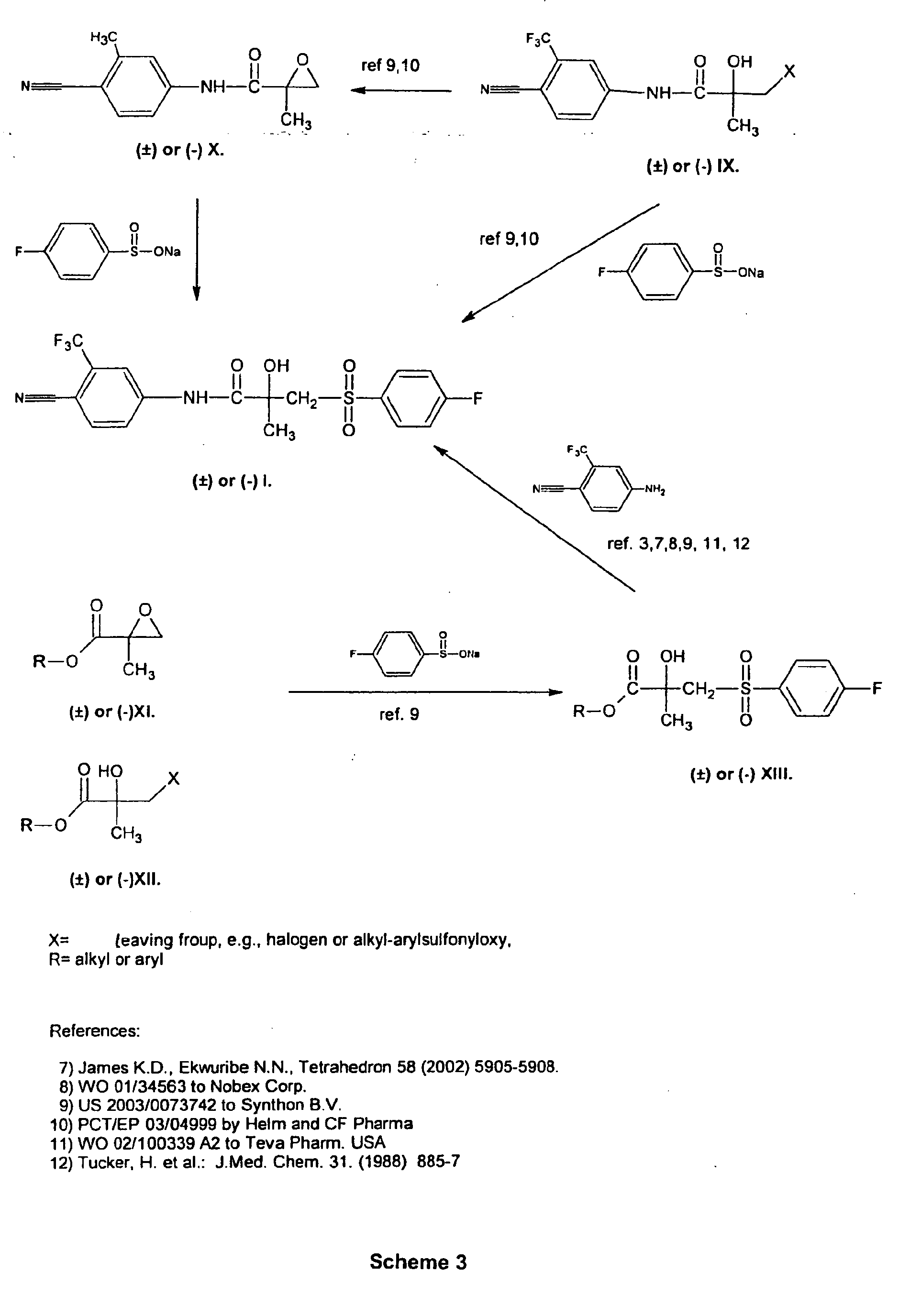

It was now found that this problem can be solved by obtaining the acid (**XIII, R=H)** 3-[(4-fluorophenyl)sulfonyl]-2-hydroxy-2-methyl propionic acid in a direct reaction with a salt of 4-fluorophenyl sulfinic acid and 3-chloro-2-methyl-2-hydroxy propionic acid (Scheme 4).

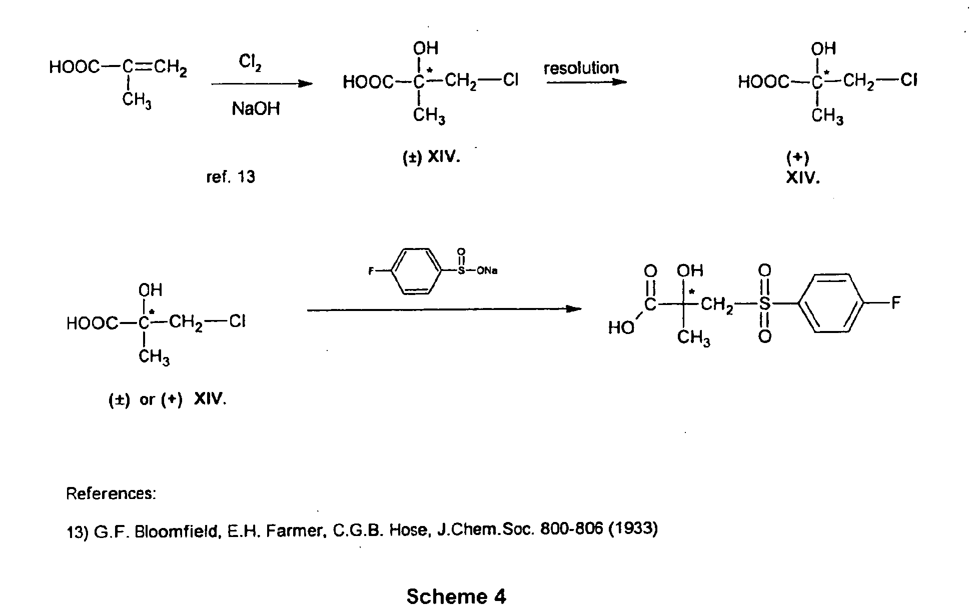

In this synthesis 3-chloro-2-methyl-2-hydroxy propionic acid (compound **XIV**) can be obtained by chlorination of methacrylic acid. Methacrylic acid is commercially available. Using the literature procedure (G.F. Bloomfield, E.H. Farmer, C.G.B. Hose, J.Chem.Soc. 800-806 (1933)) the racemic ± **XIV** chlorohydrin can be prepared by reacting hypochlorous acid with methacrylic acid. The approximately 60% optically enriched mother liquor obtained from the resolution of **XIV** with brucine can be resolved with dehydroabietylamine to obtain optically pure (+) **XIV.**

Surprisingly, the reaction yields smoothly and efficiently the desired compound **XIII** (R=H). The reaction can be conducted in organic solvents, organic solvent-water mixtures or in water in the presence or absence of phase transfer catalysts (advantageously in the presence of 2-50 mol % quaternary ammonium salt type catalyst) at a temperature between -20° and 150°C, advantageously between 20° and 110°C.

The salt of 4-fluorophenyl sulfinic acid can be selected from alkali metal, alkaline earth metal and ammonium salts. The preferred salt is the sodium salt.

Even more unexpectedly, the reaction of optically active (+)-3-chloro-2-methyl-2-hydroxy propionic acid **XIV** with 4-fluorophenyl sulfinate yielded optically active (-)-3-[4-fluorophenyl)sulfonyl]-2-hydroxy-2-methyl propionic acid. No loss of enantiomeric purity is observed under the conditions employed and utilized in the production of the target compound. 3-[(4-fluorophenyl)sulfonyl]-2-hydroxy-2-methyl propionic acid obtained in the procedure of the present invention is a crystalline compound which can be purified easily.

The high purity of the compound results in a high purity of bicalutamide synthesized therefrom. The purity is sufficiently high to further purify the compound without using complicated purification procedures that are difficult to implement in chemical plants. The characteristic contaminants of the previously known procedures could not be found in the bicalutamide prepared by the process of the present invention.

The carboxylic acid **XIII** obtained as described above is used in the acylation of 4-cyano-3-trifluoromethyl aniline. The acylation of 4-cyano-3-trifluoromethyl aniline can be accomplished in good yield by the reaction with the acid **XIII** chloride obtainable by reacting the acid **XIII** with thionyl chloride (H. Tucker, G.J. Chesterton, J.Med.Chem. **31** 885-887 (1988)).

For the acylation, however, other carboxylic group activating methods known from the art can also be used. (A description of the activating methods can be found, e.g., in Houben-Weyl: Methoden der Organischen Chemie Bd XV/2). To obtain the acyl anilide not only carboxylic, but also nitrogen activated derivatives, e.g. isocyanates can be utilized (Houben-Weyl: Methoden der Organischen Chemie Bd XV/2 183-186).

The sodium salt of the 4-fluorophenyl sulfinic acid can be prepared in good yield in simple reactions known from the literature (for example as described in Gy. Oláh; A. Pavláth: Acta Chim. Hung. **4** (1954) 111-117) or R.M. Hann, J.Am.Chem.Soc. **57** 2167-68 (1935)). In contrast to the thiophenol derivatives the sodium salt of the 4-fluorophenyl sulfinic acid is an odourless solid with high melting point whose storage and handling is not a problem.

The advantages of the process of the present invention shown in Scheme 4 compared to the known processes are as follows:
1) 3-Chloro-2-methyl-2-hydroxy propionic acid can be easily obtained from methacrylic acid by chlorination. This acid can be resolved to obtain optically active 3-chloro-2-methyl-2-hydroxy propionic acid.
2) Reaction of 3-chloro-2-methyl-2-hydroxy propionic acid with 4-fluorophenyl sulfinate directly yields 3-[(4-fluorophenyl)-2-hydroxy-2-methyl propionic acid which is a crystalline compound and can be easily purified by crystallization and can be used in the synthesis of bicalutamide without further transformation.
3) (-)-3-[(4-fluorophenyl)sulfonyl]-2-hydroxy-2-methyl propionic acid can be utilized in the synthesis of R(-)-bicalutamide.

The invention will be illustrated in more detail by the following examples, which are not intended to restrict the scope of the invention.

### Example 1

### (-)-3-Chloro-2-hydroxy-2-methyl propionic acid [(-)XIV]

53 g of racemic 3-chloro-2-hydroxy-2-methyl propionic acid (G.F. Bloomfield, E.H. Farmer, C.G.B. Hose, J.Chem.Soc. 800-806 (1933)), 100 g of brucine and 240 cm³ of water were warmed until a homogeneous solution was obtained. The solution was cooled down slowly while seeded by the brucine salt of (-)-3-chloro-2-hydroxy-2-methyl propionic acid, and the crystals precipitated were filtered at 0°C. Repetition of the crystallization yielded 44.6 g of the brucine salt of (-)-3-chloro-2-hydroxy-2-methyl propionic acid. The salt was decomposed by diluted sulfuric acid and the (-)-3-chloro-2-hydroxy-2-methyl propionic acid was extracted with ethyl acetate and after evaporation of the solvent then the optically active chlorohydrin was recrystallized from toluene to yield 4.8 g (-)-3-chloro-2-hydroxy-2-methyl propionic acid which has an optical rotation of -14.3° (c=1, methanol, λ=589 nm, I=1 dm). 25.0 g of chlorohydrin enriched in (+)-enantiomer was obtained from the brucine salt obtained from the first crystallization's evaporated mother liquor which was decomposed by acidification with hydrochloric acid, then the water phase was extracted several times with ethyl acetate and evaporation of the solvent ([α]=+8.0° (c=1, methanol, λ=589 nm, I=1 dm), m.p. 106-108°C.

### Example 2

### (+)-3-Chloro-2-hydroxy-2-methyl propionic acid [(+)XIV]

3 g of chlorohydrin enriched in the (+)-isomer obtained as described in Example 1 and characterized by the specific rotation of +8.0° (c=1, methanol, λ=589 nm, I=1 dm) is dissolved in 12 cm³ of methanol and the solution of 4.7 g of dehydroabietylamine in 5 cm³ methanol was added. The crystals were filtered and recrystallized two times from methanol. Decomposing the dehydroabietylamine salt as described in Example 1 1.6 g of (+)-chlorohydrin was obtained which was recrystallized from methanol to yield 1.4 g of (+)-3-chloro-2-hydroxy-2-methyl propionic acid with a specific rotation of [α]=+14.5° (c=1, methanol, λ=589 nm, I=1 dm), m.p. 107-109°C.

### Example 3

### (-)-3-(4-Fluoro-benzenesulfonyl)-2-hydroxy-2-methyl propionic acid

1.38 g (10 mmol) of (+)-2-hydroxy-2-methyl-3-chloro propionic acid ((+)**XIV**, Example 2), 2.91 g (16 mmol) of sodium 4-fluorophenyl-sulfinate, 5 g of sodium carbonate, 5 cm³ of water and 1.0 g tetrabutyl ammonium chloride was placed into a round-bottomed flask. After refluxing the reaction mixture for 24 hours it was diluted with 30 cm³ of water, cooled to ambient temperature and the pH of the reaction mixture was adjusted to pH=3 by careful addition of concentrated hydrochloric acid.

The reaction mixture was extracted with 6 x 50 cm³ of ethyl acetate, the combined organic phases were dried and evaporated, finally the evaporation residue was purified by refluxing with dichloromethane.

Yield: 1.2 g (46%). Specific rotation: [α]=-3.3° (c=1, methanol, λ=589 nm, I=1 dm). Melting point: 125-126°C.

### Example 4

### (±)-3-(4-Fluoro-benzenesulfonyl)-2-hydroxy-2-methyl propionic acid

Reproducing the procedure described in Example 3 the racemic compound **[(±)XIII,** R=H] was obtained from the racemic (±)**XIV**. Yield: 1.36 g (52%), melting point: 123-125°C.

## Claims

1. Process for the preparation of 3-[(4-fluorophenyl)sulfonyl]-2-hydroxy-2-methyl propionic acid, which comprises the step of reacting 3-chloro-2-methyl-2-hydroxy propionic acid with a salt of 4-fluorophenyl sulfinic acid.

2. Process according to claim 1, wherein the salt of 4-fluorophenyl sulfinic acid is selected from the group consisting of alkali, alkaline earth and ammonium salts, preferably sodium salt.

3. Process according to any of the preceding claims, wherein (±)- or (-)-3-[(4-fluorophenyl)sulfonyl]-2-hydroxy-2-methyl propionic acid is obtained.

4. Process according to any of the preceding claims, wherein the 3-chloro-2-methyl-2-hydroxy propionic acid is employed essentially as (+)-3-chloro-2-methyl-2-hydroxy propionic acid.

5. Process for the preparation of bicalutamide, which comprises the step of any of the preceding claims.

6. Use of (±)- or (-)-3-[(4-fluorophenyl)sulfonyl]-2-hydroxy-2-methyl propionic acid for the preparation bicalutamide.

7. Use of (±)- or (+)-3-chloro-2-methyl-2-hydroxy propionic acid for the preparation of bicalutamide.

8. (±)- or (-)-3-[(4-fluorophenyl)sulfonyl]-2-hydroxy-2-methyl propionic acid.

9. (+)-3-chloro-2-methyl-2-hydroxy propionic acid.
